# EUROPEAN PATENT SPECIFICATION

(11) **EP 4 311 547 B1**
(45) Date of publication and mention of the grant of the patent: **14.10.2026**
(21) Application number: 22763403.7
(22) Date of filing: 03.03.2022
(51) Int. Cl.: A61K 31/192, A61K 9/70, A61K 47/02, A61K 47/06, A61K 47/10, A61K 47/12, A61K 47/14, A61K 47/32, A61K 47/44, A61P 29/00

(54) **LOXOPROFEN-CONTAINING ADHESIVE PATCH AND METHOD FOR MANUFACTURING SAME**
LOXOPROFENHALTIGES KLEBEPFLASTER UND VERFAHREN ZUR HERSTELLUNG DAVON
PATCH ADHÉSIF CONTENANT DU LOXOPROFÈNE ET PROCÉDÉ DE FABRICATION D'UN TEL PATCH

(30) Priority: 04.03.2021 JP 2021034394
(43) Date of publication of application: 31.01.2024
(73) Proprietor: Teikoku Seiyaku Co., Ltd., Higashikagawa-shi, Kagawa 769-2695 (JP)
(72) Inventor: HARAUCHI, Yuki, Higashikagawa-shi, Kagawa 769-2695 (JP)
(74) Representative: Kraus & Lederer PartGmbB
(86) International application number: PCT/JP2022/009210
(87) International publication number: WO 2022/186351

(56) References cited:
- WO-A1-2013/008909
- WO-A1-2013/008909
- WO-A1-2019/160067
- WO-A1-2019/160067
- JP-A- 2002 226 366
- JP-A- 2011 020 997
- JP-A- 2019 055 928
- JP-B1- 5 450 910

## Description

### TECHNICAL FIELD

The present invention relates to a patch comprising loxoprofen or a salt thereof, or a hydrate thereof, lactic acid as the sole hydroxy acid, and zinc oxide as the sole metal oxide, and a method for producing the same.

### BACKGROUND ART

Loxoprofen is one of the nonsteroidal anti-inflammatory analgesics that exhibits an excellent anti-inflammatory analgesic effect, and has been used as an external agent for a long time, and various studies have been conducted so far to improve the stability and skin permeability of the drug. For example, a technique that suppresses the esterification reaction of a non-steroidal anti-inflammatory analgesic having a carboxy group in the molecule with L-menthol by adding a metal oxide (Patent Document 1), and a technique for suppressing the generation of related substances and improving the drug release rate into the skin by adding an anti-inflammatory drug component having a carboxy group, a C12 to C18 fatty acid, a metal oxide, and liquid paraffin in a pasty preparation, and using a monohydric lower alcohol at the time of production (Patent Document 2), are disclosed. However, these documents do not specifically disclose a loxoprofen-containing patch. Also, regarding a patch containing loxoprofen sodium, a technique for suppressing the generation of degradation products and improving permeability by adding lactic acid and a higher fatty acid at a specific ratio is disclosed (Patent Document 3). However, the patch and the method for producing the same of the present invention are not disclosed nor suggested.

### CITATION LIST

### PATENT DOCUMENT

Patent Document 1: JP 2002-226366 A
Patent Document 2: WO 2019/160067 pamphlet
Patent Document 3: WO 2013/008909 pamphlet

JP 5450910 B1 relates to provision of the pharmaceutical composition by which interaction between a loxoprofen or its salt, and an interaction component was inhibited.
WO 2013/ 008909 A1 provides loxoprofen sodium-containing adhesive patch for external applications, which has high transdermal absorbability of the main ingredient contained therein and has high stability so that the production of any decomposition product can be prevented during storage.
WO 2019/ 160067 A1 relates to relates to a non-aqueous skin patch and a method for manufacturing the same, the non-aqueous skin patch comprising: a medicinal component including an anti-inflammatory drug having a carboxyl group in a plaster; a C12-C18 fatty acid; a metal oxide; and a liquid paraffin.

### SUMMARY OF INVENTION

### TECHNICAL PROBLEM

An object of the present invention is to provide a patch that suppresses the degradation products of loxoprofen and shows excellent skin permeability, and a method for producing the same.

### SOLUTION TO PROBLEM

The present inventor has earnestly studied in order to solve the above problems. As a result, he has surprisingly found that a patch comprising loxoprofen or a salt thereof, or a hydrate thereof (hereinafter also referred to as "loxoprofen component"), lactic acid as the sole hydroxy acid, and zinc oxide as the sole metal oxide in a pasty preparation not only suppresses the generation of the menthol ester form in case L-menthol is added, but also suppresses the generation of the ring-opened form in which the 5 membered ring (cyclopentyl ring) of loxoprofen is opened (hereinafter referred to as "ring-opened form of loxoprofen") which is an oxidative degradation product, and finally completed the present invention. Further, the present inventor has also found that the stability and skin permeability of the drug of said patch are improved by adding additives at the production of a drug liquid in a specific order. More specifically, the present inventor has found that when a drug liquid comprising a loxoprofen component, lactic acid as the sole hydroxy acid, and zinc oxide as the sole metal oxide is prepared, if the hydroxy acid and the loxoprofen component is mixed at first and then the metal oxide is added thereto, the workability of the preparation of the drug liquid can be improved, and further a patch that suppresses the generation of related substances as well as shows excellent skin permeability can be obtained.

The present invention provides a patch characterized by comprising a loxoprofen component, lactic acid as the sole hydroxy acid, and zinc oxide as the sole metal oxide in a pasty preparation, and a method for producing the same.

Namely, the present disclosure relates to the followings.
[1] A patch comprising loxoprofen or a salt thereof, or a hydrate thereof; a C₂-C₆ hydroxy acid; and a metal oxide in a pasty preparation (hereinafter also referred to as "Present patch").
[2] The patch according to [1], wherein
   the loxoprofen or a salt thereof, or a hydrate thereof is loxoprofen sodium or a hydrate thereof;
   the C₂-C₆ hydroxy acid is one or more selected from the group consisting of glycolic acid, lactic acid, glyceric acid, 2-hydroxybutyric acid, 3-hydroxybutyric acid, γ-hydroxybutyric acid, malic acid, tartaric acid, citric acid, isocitric acid, mevalonic acid, and pantoic acid; and
   the metal oxide is one or more selected from the group consisting of zinc oxide, magnesium oxide, aluminum oxide, calcium oxide, titanium oxide, and iron oxide.
[3] The patch according to [1]or [2], wherein
   the loxoprofen or a salt thereof, or a hydrate thereof is loxoprofen sodium dihydrate;
   the C₂-C₆ hydroxy acid is lactic acid; and
   the metal oxide is zinc oxide.
[4] The patch according to any one of [1] to [3], wherein the contained amount of the loxoprofen or a salt thereof, or a hydrate thereof is 0.1 to 10% by weight, the contained amount of the C₂-C₆ hydroxy acid is 0.1 to 2.0% by weight, and the contained amount of the metal oxide is 0.03 to 1.5% by weight, relative to the pasty preparation weight.
[5] The patch according to any one of [1] to [4], wherein the weight ratio of the contained amount of the loxoprofen or a salt thereof, or a hydrate thereof and the contained amount of the C₂-C₆ hydroxy acid is 1 : 0.01 to 1 : 0.4.
[6] The patch according to any one of [1] to [5], wherein the weight ratio of the contained amount of the loxoprofen or a salt thereof, or a hydrate thereof and the contained amount of the metal oxide is 1 : 0.03 to 1 : 0.3.
[7] The patch according to any one of [1] to [6] further comprising one or more ingredient(s) selected from the group consisting of a base polymer, a tackifier resin, and a softener in the pasty preparation.
[8] The patch according to [7], wherein
   the base polymer is a styrene-isoprene-styrene block copolymer;
   the tackifier resin is an alicyclic saturated hydrocarbon resin; and
   the softener is one or more selected from the group consisting of polybutene and liquid paraffin.
[9] The patch according to [7]or [8], wherein the contained amount of the base polymer is 5 to 50% by weight, the contained amount of the tackifier resin is 10 to 60% by weight, and the contained amount of the softener is 10 to 60% by weight, relative to the pasty preparation weight.
[10] The patch according to any one of [1] to [9] further comprising one or more ingredient(s) selected from the group consisting of L-menthol, a higher fatty acid, a fatty acid ester, and an antioxidant in the pasty preparation.
[11] The patch according to [10], wherein
   the higher fatty acid is isostearic acid;
   the fatty acid ester is diethyl sebacate; and
   the antioxidant is dibutylhydroxytoluene.
[12] The patch according to [10]or [11], wherein the contained amount of the L-menthol is 0.1 to 10% by weight, the contained amount of the higher fatty acid is 0.1 to 20% by weight, the contained amount of the fatty acid ester is 0.1 to 20% by weight, and the contained amount of the antioxidant is 0.05 to 5% by weight, relative to the pasty preparation weight.
[13] The patch according to any one of [1] to [10] comprising loxoprofen sodium dihydrate, lactic acid, zinc oxide, a styrene-isoprene-styrene block copolymer, an alicyclic saturated hydrocarbon resin, polybutene, liquid paraffin, L-menthol, isostearic acid, and dibutylhydroxytoluene in a pasty preparation.
[14] A method for producing the patch according to any one of [1] to [13], comprising
   Step 1: dissolving loxoprofen or a salt thereof, or a hydrate thereof in a C₂-C₆ hydroxy acid-containing solution to obtain a loxoprofen-containing solution; and
   Step 2: mixing the loxoprofen-containing solution obtained in Step 1 and a metal oxide to obtain a drug liquid (hereinafter also referred to as "Present production method").
[15] The method according to [14], wherein the C₂-C₆ hydroxy acid-containing solution comprises a C₂-C₆ hydroxy acid and one or more ingredient(s) selected from the group consisting of L-menthol, a higher fatty acid, and a fatty acid ester.

### EFFECT OF INVENTION

According to the present invention, a patch that suppresses the generation of degradation products of loxoprofen and shows excellent skin permeability, and a method for producing the same in which the workability at the production is improved, can be provided.

### DESCRIPTION OF EMBODIMENTS

In the present description, "contain" or "comprise" may be used interchangeably with "blend". Also, in the present description, "contained amount" may be used interchangeably with "amount".

The Present patch comprises a loxoprofen component, a lactic acid as the sole hydroxy acid, and zinc oxide as the sole metal oxide in a pasty preparation. The pasty preparation is a pasty composition usually comprising an adhesive base comprising an adhesive as a main ingredient, and a drug, and may also be referred to as "adhesive composition".

Examples of the loxoprofen component contained in the Present patch as an active ingredient include a free form of loxoprofen, a base addition salt of loxoprofen (for example, loxoprofen sodium), and a hydrate of a free form of loxoprofen or a base addition salt of loxoprofen (for example, loxoprofen sodium dihydrate). As the loxoprofen component, loxoprofen sodium or a hydrate thereof is preferable, and loxoprofen sodium dihydrate is more preferable.

The contained amount of the loxoprofen component is not specifically limited as long as formulating can be carried out, but is usually within a range of 0.1 to 10% by weight, preferably 0.5 to 8% by weight, and more preferably 1 to 6% by weight, relative to the pasty preparation weight. When the contained amount of the loxoprofen component in the pasty preparation is less than 0.1% by weight, the transdermal absorbability becomes insufficient. Meanwhile, when said amount is more than 10% by weight, the physical properties of the patch are impaired and said amount is also economically disadvantageous.

The hydroxy acid contained in the Present patch is a very excellent solubilizer of the loxoprofen component, and the loxoprofen component can be stably dissolved in the adhesive base and an excellent transdermal absorption promoting effect can be achieved by containing it.

The lactic acid as the sole hydroxy acid contained in the Present patch is not specifically limited as long as it is an acid with 2 to 6 carbon atoms having hydroxy group(s) and carboxy group(s) in the molecule. Examples of the hydroxy acid include one or more selected from the group consisting of glycolic acid, glyceric acid, 2-hydroxybutyric acid, 3-hydroxybutyric acid, γ-hydroxybutyric acid, malic acid, tartaric acid, citric acid, isocitric acid, mevalonic acid, and pantoic acid, one or more selected from the group consisting of glycolic acid, glyceric acid, 2-hydroxybutyric acid, 3-hydroxybutyric acid, γ-hydroxybutyric acid, malic acid, and tartaric acid.

The hydroxy acid is an additive which may react with the loxoprofen component, produce degradation products thereof, and decrease the stability of the formulation. Thus, the contained amount of the hydroxy acid in the Present patch is usually within a range of 0.1 to 2.0% by weight, preferably 0.1 to 1.7% by weight, and more preferably 0.1 to 1.5% by weight, relative to the pasty preparation weight. When the contained amount of the hydroxy acid is less than 0.1% by weight, it is impossible to sufficiently dissolve the loxoprofen component in the adhesive base, and decrease in the transdermal absorbability of the formulation, crystal precipitation of the drug in the formulation during the storage, or the like may occur. Meanwhile, when the contained amount of the hydroxy acid is more than 4.0% by weight, degradation product(s) derived from the hydroxy acid increases, and the stability of the formulation decreases.

Also, the weight ratio of the contained amount of the loxoprofen component and the contained amount of the hydroxy acid is preferably 1 : 0.01 to 1 : 0.4, more preferably 1 : 0.05 to 1 : 0.35, and further preferably 1 : 0.07 to 1 : 0.3.

The zinc oxide as the sole metal oxide contained in the Present patch has a function that suppresses the generation of degradation products of loxoprofen.

Examples of the metal oxide include one or more selected from the group consisting of magnesium oxide, aluminum oxide, calcium oxide, titanium oxide, and iron oxide.

The amount of the metal oxide contained in the Present patch is usually within a range of 0.03 to 1.5% by weight, preferably 0.06 to 1.2% by weight, and more preferably 0.1 to 1.0% by weight, relative to the pasty preparation weight.

The amount of the metal oxide contained in the Present patch is preferably adjusted depending on the contained amount of the loxoprofen component in order to suppress the generation of degradation products of the loxoprofen component. The weight ratio of the contained amount of the loxoprofen component and the contained amount of the metal oxide is preferably within a range of 1 : 0.03 to 1 : 0.3, more preferably 1 : 0.04 to 1 : 0.3, and further preferably 1 : 0.05 to 1 : 0.3. When the weight ratio of the contained amount of the metal oxide relative to the loxoprofen component is less than 0.03, the effect for suppressing the generation of degradation products becomes insufficient. Meanwhile, when the ratio is more than 0.3, the skin permeability of the drug is suppressed.

Also, the metal oxide is not dissolved, but dispersed in the formulation. The metal oxide contained in the Present patch preferably has a particle size of 1 to 100 nm in view of the dispersibility at the time of manufacture.

In one embodiment, the pasty preparation of the Present patch may further comprise one or more ingredient(s) selected from the group consisting of a base polymer, a tackifier resin, and a softener, and may further optionally comprise other ingredient(s).

Examples of the base polymer which can be used in the Present patch include acrylic adhesives, rubber adhesives, and silicone adhesives, and a rubber adhesive is preferably used. Examples of the rubber adhesive include a natural rubber, polyisobutylene, polyisoprene, polybutadiene, a styrene-isoprene-styrene block copolymer (hereinafter also referred to as "SIS"), a styrene-butadiene rubber, and a styrene-isoprene rubber. One of them or a combination of two or more of them may be used, and a styrene-isoprene-styrene block copolymer (SIS) is preferably used.

The amount of the base polymer contained in the Present patch is determined in view of the contained amounts of other ingredients, and usually within a range of 5 to 50% by weight, preferably 10 to 40% by weight, and more preferably 10 to 30% by weight, relative to the pasty preparation weight. When the contained amount of the base polymer in the pasty preparation is less than 5% by weight, the cohesive force and/or shape retention property of the pasty preparation decrease(s). Meanwhile, when the amount is more than 50% by weight, the adhesive force decreases, the pasty preparation becomes heterogeneous, and the workability during the manufacture decreases.

The tackifier resin used in the Present patch is not specifically limited as long as it is generally contained in a patch, and examples thereof include a rosin resin such as a rosin ester and a hydrogenated rosin glycerol ester; a petroleum resin such as an alicyclic saturated hydrocarbon resin; and a terpene resin, and one of them or a combination of two or more of them may be used. One or more selected from the group consisting of a rosin resin and a petroleum resin are preferable, one or more selected from the group consisting of a hydrogenated rosin glycerol ester and an alicyclic saturated hydrocarbon resin are more preferable, and an alicyclic saturated hydrocarbon resin is further preferable.

The amount of the tackifier resin contained in the Present patch is not specifically limited as long as formulating can be carried out, but is usually within a range of 10 to 60% by weight, preferably 15 to 50% by weight, and more preferably 20 to 40% by weight, relative to the pasty preparation weight. When the contained amount of the tackifier resin in the pasty preparation is less than 10% by weight, the physical properties of the formulation are deteriorated and undesirable phenomena such as pasty preparation residues at the peeling occur. Meanwhile, when said amount is more than 60% by weight, skin irritation occurs.

The softener used in the Present patch is not specifically limited as long as it is compatible with the other base ingredients and provides the pasty preparation with plasticity. Examples of the softener which can be used in the Present patch include polyisobutylene, liquid polyisoprene, polybutene, lanolin, castor oil, almond oil, olive oil, camellia oil, persic oil, peanut oil, process oil, extender oil, and liquid paraffin, and one of them or a combination of two or more of them may be used. One or more selected from the group consisting of polybutene and liquid paraffin are preferable.

The amount of the softener contained in the Present patch is determined also in view of the contained amounts of the other liquid ingredients contained in the pasty preparation, and usually within a range of 10 to 60% by weight, preferably 20 to 50% by weight, and more preferably 30 to 45% by weight, relative to the pasty preparation weight.

In one embodiment, the pasty preparation of the Present patch may further comprise L-menthol.

L-menthol contained in the Present patch functions as a transdermal absorption enhancer and a refreshing agent.

The amount of the L-menthol contained in the Present patch is not specifically limited as long as formulating can be carried out, but is usually within a range of 0.1 to 10% by weight, preferably 0.5 to 8% by weight, and more preferably 1 to 5% by weight, relative to the pasty preparation weight. When the contained amount of the L-menthol in the pasty preparation is less than 0.1% by weight, the transdermal absorbability becomes insufficient. Meanwhile, when said amount is more than 10% by weight, the physical properties of the patch are impaired.

In one embodiment, the pasty preparation of the Present patch may further comprise liquid ingredient(s). In this regard, the liquid ingredient is not necessarily liquid at the normal temperature, and those which are converted to liquids by heating at the time of manufacture are also encompassed.

The liquid ingredient contained in the Present patch has a function that improves the manufacturability by adjusting the viscosity of the drug liquid. Examples of such liquid ingredient include higher fatty acids and fatty acid esters.

Examples of the higher fatty acid include isostearic acid, myristic acid, palmitic acid, stearic acid, oleic acid, linoleic acid, linolenic acid, capric acid, lauric acid, and caproic acid, and one of them or a combination of two or more of them may be used. One or more selected from the group consisting of isostearic acid and oleic acid are preferable, and isostearic acid is more preferable.

The amount of the higher fatty acid contained in the Present patch is usually within a range of 0.1 to 20% by weight, preferably 0.5 to 15% by weight, and more preferably 1 to 10% by weight, relative to the pasty preparation weight.

Examples of the fatty acid ester include hexyl laurate, isopropyl myristate, methyl myristate, myristyl myristate, cetyl myristate, octyldodecyl myristate, isopropyl palmitate, cetyl palmitate, isopropyl isostearate, diisobutyl adipate, diisopropyl adipate, dioctyl adipate, diisopropyl sebacate, diethyl sebacate, ethyl oleate, decyl oleate, oleyl oleate, ethyl linoleate, isopropyl linoleate, cetyl lactate, and ethyl lactate, and one of them or a combination of two or more of them may be used. One or more selected from the group consisting of isopropyl myristate, isopropyl palmitate, isopropyl isostearate, diisopropyl adipate, and diethyl sebacate are preferable, and diethyl sebacate is more preferable.

The amount of the fatty acid ester contained in the Present patch is appropriately adjusted by the viscosity of the drug liquid within a range which does not affect the formulation properties, and usually within a range of 0.1 to 20% by weight, preferably 0.5 to 15% by weight, and more preferably 1 to 10% by weight, relative to the pasty preparation weight.

Also, the Present patch may comprise various base ingredients used in normal patches as long as they do not affect the other ingredients. Examples of such base ingredients include, but are not specifically limited to, cellulose derivatives such as polyvinylpyrrolidone, polyvinyl alcohol, and hydroxypropyl methylcellulose; silicon compounds such as anhydrous silicic acid and light anhydrous silicic acid; antioxidants such as dibutylhydroxytoluene (also referred to as "BHT"), pentaerythrityl-tetrakis-[3-(3,5-di-t-butyl-4-hydroxyphenyl)propionate], tocopherol acetate, and ascorbic acid; and inorganic fillers such as silica and zinc stearate. The Present patch may further comprise preservatives such as p-hydroxybenzoates (for example, methyl p-hydroxybenzoate); fungicides such as ethanol and isopropyl alcohol; flavoring agents such as mentha oil; colorants such as yellow ferric oxide, and the like, if necessary, and they may be optionally contained in the Present patch at appropriate amounts.

In one embodiment, the pasty preparation of the Present patch may further comprise an antioxidant. As the antioxidant, dibutylhydroxytoluene is preferable.

The amount of the antioxidant contained in the Present patch is usually within a range of 0.05 to 5% by weight, preferably 0.1 to 1% by weight, and more preferably 0.2 to 0.5% by weight, relative to the pasty preparation weight.

The Present patch usually consists of a backing, a pasty preparation, and a release liner, and the pasty preparation is spread or applied between a backing and a release liner.

Examples of the backing used in the Present patch include films, non-woven fabrics, woven fabrics, knitted fabrics, and laminate composites of non-woven fabrics and films. Examples of the material of these backings include polyethylene, polypropylene, polyvinyl chloride, polyester, polyethylene terephthalate, nylon, polyurethane, rayon, polyacrylonitrile, polystyrene, and polyethylene naphthalate.

Examples of the release liner used in the Present patch include polyethylene terephthalate, polypropylene, and paper, and especially polyethylene terephthalate is preferable. The release liner may be siliconized if necessary in order to optimize the release force.

Examples of the production method of the Present patch include, but are not limited to, a solvent method characterized in that the drug and each base ingredient are dissolved in an organic solvent such as toluene and hexane, and the organic solvent is removed by coating and drying steps; and a hot melt method characterized in that an adhesive composition is melted at a high temperature of 100°C or higher, and then a coating step is carried out. In one embodiment, the Present patch is produced by a hot melt method.

In the present description, the term of "C₂-C₆ hydroxy acid-containing solution" may be a solution comprising a C₂-C₆ hydroxy acid only, or a solution comprising a C₂-C₆ hydroxy acid and other ingredient(s) (for example, one or more ingredient(s) selected from the group consisting of L-menthol, a higher fatty acid, and a fatty acid ester). Examples of the C₂-C₆ hydroxy acid-containing solution include a lactic acid solution, and a solution consisting of lactic acid and one or more ingredient(s) selected from the group consisting of L-menthol, a higher fatty acid, and a fatty acid ester.

In the present description, the term of "loxoprofen-containing solution" means a solution obtained by dissolving a loxoprofen component in a C₂-C₆ hydroxy acid-containing solution.

In the present description, the term of "drug liquid" means a liquid obtained by mixing a loxoprofen-containing solution and a metal oxide.

In the present description, the term of "adhesive solution" means a solution obtained by mixing ingredients comprising an adhesive other than a drug liquid.

The method for producing the Present patch comprises
Step 1: dissolving a loxoprofen component in a C₂-C₆ hydroxy acid-containing solution to obtain a loxoprofen-containing solution; and
Step 2: mixing the loxoprofen-containing solution obtained in Step 1 and a metal oxide to obtain a drug liquid.

In one embodiment of the Present production method, the C₂-C₆ hydroxy acid-containing solution comprises a C₂-C₆ hydroxy acid and one or more ingredient(s) selected from the group consisting of L-menthol, a higher fatty acid, and a fatty acid ester.

Another embodiment of the Present production method further comprises
Step 3: mixing one or more other ingredient(s) selected from the group consisting of a base polymer, a tackifier resin, a softener, and an antioxidant to obtain an adhesive solution;
Step 4: mixing the drug liquid obtained in Step 2 and the adhesive solution obtained in Step 3 to obtain an adhesive composition;
Step 5: coating a release liner with the adhesive composition obtained in Step 4 to form an adhesive layer; and
Step 6: applying the adhesive layer obtained in Step 5 on a backing to obtain a laminate.

Because the Present patch comprises loxoprofen as an active ingredient, it is useful in the prevention or treatment of disease(s) or symptom(s) whose clinical condition(s) is/are expected to be improved by administering loxoprofen. Examples of such disease(s) or symptom(s) include inflammation and pain. Also, examples of the inflammation or pain include muscle pain, osteoarthritis, and posttraumatic swelling and pain.

In the present invention, the term of "prevention" means administering the Present patch to an individual who has not developed disease(s) or symptom(s). Also, the term of "treatment" means administering the Present patch to an individual who has already developed disease(s) or symptom(s). Thus, administering the Present patch to an individual who has already developed disease(s) or symptom(s) for the prevention of deterioration, attack, or recurrence of symptom(s) and the like is an aspect of "treatment".

The Present patch is usually applied to a patient, for example a human or an animal, and preferably a human who suffers from or is at risk of suffering from the above disease(s) or symptom(s). The frequency of application may appropriately vary depending on conditions such as severity of the disease(s) or symptom(s), age, weight, and sex of the patient, and the amount of loxoprofen in patch. When applied to a human, the Present patch is usually exchanged one or more time(s) per day such as 1 to 3 time(s), 1 to 2 time(s), and once per day, or every several days such as every 2 to 3 days.

Hereinafter, aspects of the present invention are described. Aspects produced by optionally combining the following aspects and aspects produced by optionally combining the following aspect(s) with any of aspect(s) or embodiment(s) etc. disclosed in the present description are also encompassed by the present invention.

### 1. Patch

### [Aspect 1]

The Present patch comprising a loxoprofen component, a C₂-C₆ hydroxy acid, and a metal oxide in a pasty preparation.

### [Aspect 2]

The Present patch according to the Aspect 1, wherein the loxoprofen component is loxoprofen sodium or a hydrate thereof.

### [Aspect 3]

The Present patch according to the Aspect 1, wherein the loxoprofen component is loxoprofen sodium dihydrate.

### [Aspect 4]

The Present patch according to any one of the Aspects 1 to 3, wherein the C₂-C₆ hydroxy acid is one or more selected from the group consisting of glycolic acid, lactic acid, glyceric acid, 2-hydroxybutyric acid, 3-hydroxybutyric acid, γ-hydroxybutyric acid, malic acid, tartaric acid, citric acid, isocitric acid, mevalonic acid, and pantoic acid.

### [Aspect 5]

The Present patch according to any one of the Aspects 1 to 3, wherein the C₂-C₆ hydroxy acid is one or more selected from the group consisting of glycolic acid, lactic acid, glyceric acid, 2-hydroxybutyric acid, 3-hydroxybutyric acid, γ-hydroxybutyric acid, malic acid, and tartaric acid.

### [Aspect 6]

The Present patch according to any one of the Aspects 1 to 3, wherein the C₂-C₆ hydroxy acid is lactic acid.

### [Aspect 7]

The Present patch according to any one of the Aspects 1 to 6, wherein the metal oxide is one or more selected from the group consisting of zinc oxide, magnesium oxide, aluminum oxide, calcium oxide, titanium oxide, and iron oxide.

### [Aspect 8]

The Present patch according to any one of the Aspects 1 to 6, wherein the metal oxide is one or more selected from the group consisting of zinc oxide, magnesium oxide, aluminum oxide, and calcium oxide.

### [Aspect 9]

The Present patch according to any one of the Aspects 1 to 6, wherein the metal oxide is zinc oxide.

### [Aspect 10]

The Present patch according to the Aspect 1, wherein
the loxoprofen or a salt thereof, or a hydrate thereof is loxoprofen sodium or a hydrate thereof;
the C₂-C₆ hydroxy acid is one or more selected from the group consisting of glycolic acid, lactic acid, glyceric acid, 2-hydroxybutyric acid, 3-hydroxybutyric acid, γ-hydroxybutyric acid, malic acid, tartaric acid, citric acid, isocitric acid, mevalonic acid, and pantoic acid; and
the metal oxide is one or more selected from the group consisting of zinc oxide, magnesium oxide, aluminum oxide, calcium oxide, titanium oxide, and iron oxide.

### [Aspect 11]

The Present patch according to the Aspect 1, wherein
the loxoprofen or a salt thereof, or a hydrate thereof is loxoprofen sodium dihydrate;
the C₂-C₆ hydroxy acid is lactic acid; and
the metal oxide is zinc oxide.

### [Aspect 12]

The Present patch according to any one of the Aspects 1 to 11, wherein the contained amount of the loxoprofen component is 0.1 to 10% by weight, the contained amount of the C₂-C₆ hydroxy acid is 0.1 to 2.0% by weight, and the contained amount of the metal oxide is 0.03 to 1.5% by weight, relative to the pasty preparation weight.

### [Aspect 13]

The Present patch according to any one of the Aspects 1 to 11, wherein the contained amount of the loxoprofen component is 0.5 to 8% by weight, the contained amount of the C₂-C₆ hydroxy acid is 0.1 to 1.7% by weight, and the contained amount of the metal oxide is 0.06 to 1.2% by weight, relative to the pasty preparation weight.

### [Aspect 14]

The Present patch according to any one of the Aspects 1 to 11, wherein the contained amount of the loxoprofen component is 1 to 6% by weight, the contained amount of the C₂-C₆ hydroxy acid is 0.1 to 1.5% by weight, and the contained amount of the metal oxide is 0.1 to 1.0% by weight, relative to the pasty preparation weight.

### [Aspect 15]

The Present patch according to any one of the Aspects 1 to 14, wherein the weight ratio of the contained amount of the loxoprofen component and the contained amount of the C₂-C₆ hydroxy acid is 1 : 0.01 to 1 : 0.4.

### [Aspect 16]

The Present patch according to any one of the Aspects 1 to 14, wherein the weight ratio of the contained amount of the loxoprofen component and the contained amount of the C₂-C₆ hydroxy acid is 1 : 0.05 to 1 : 0.35.

### [Aspect 17]

The Present patch according to any one of the Aspects 1 to 14, wherein the weight ratio of the contained amount of the loxoprofen component and the contained amount of the C₂-C₆ hydroxy acid is 1 : 0.07 to 1 : 0.3.

### [Aspect 18]

The Present patch according to any one of the Aspects 1 to 17, wherein the weight ratio of the contained amount of the loxoprofen component and the contained amount of the metal oxide is 1 : 0.03 to 1 : 0.3.

### [Aspect 19]

The Present patch according to any one of the Aspects 1 to 17, wherein the weight ratio of the contained amount of the loxoprofen component and the contained amount of the metal oxide is 1 : 0.04 to 1 : 0.3.

### [Aspect 20]

The Present patch according to any one of the Aspects 1 to 17, wherein the weight ratio of the contained amount of the loxoprofen component and the contained amount of the metal oxide is 1 : 0.05 to 1 : 0.3.

### [Aspect 21]

The Present patch according to any one of the Aspects 1 to 20, wherein the particle size of the metal oxide is 1 to 100 nm.

### [Aspect 22]

The Present patch according to any one of the Aspects 1 to 21 further comprising one or more ingredient(s) selected from the group consisting of a base polymer, a tackifier resin, and a softener in the pasty preparation.

### [Aspect 23]

The Present patch according to the Aspect 22, wherein the base polymer is a rubber adhesive.

### [Aspect 24]

The Present patch according to the Aspect 23, wherein the rubber adhesive is one or more selected from the group consisting of a natural rubber, polyisobutylene, polyisoprene, polybutadiene, a styrene-isoprene-styrene block copolymer, a styrene-butadiene rubber, and a styrene-isoprene rubber.

### [Aspect 25]

The Present patch according to the Aspect 23, wherein the rubber adhesive is a styrene-isoprene-styrene block copolymer.

### [Aspect 26]

The Present patch according to any one of the Aspects 22 to 25, wherein the tackifier resin is one or more selected from the group consisting of a rosin resin and a petroleum resin.

### [Aspect 27]

The Present patch according to any one of the Aspects 22 to 25, wherein the tackifier resin is one or more selected from the group consisting of a hydrogenated rosin glycerol ester and an alicyclic saturated hydrocarbon resin.

### [Aspect 28]

The Present patch according to any one of the Aspects 22 to 25, wherein the tackifier resin is an alicyclic saturated hydrocarbon resin.

### [Aspect 29]

The Present patch according to any one of the Aspects 22 to 28, wherein the softener is one or more selected from the group consisting of polyisobutylene, liquid polyisoprene, polybutene, lanolin, castor oil, almond oil, olive oil, camellia oil, persic oil, peanut oil, process oil, extender oil, and liquid paraffin.

### [Aspect 30]

The Present patch according to any one of the Aspects 22 to 28, wherein the softener is one or more selected from the group consisting of polybutene and liquid paraffin.

### [Aspect 31]

The Present patch according to the Aspect 22, wherein
the base polymer is a styrene-isoprene-styrene block copolymer;
the tackifier resin is an alicyclic saturated hydrocarbon resin; and
the softener is one or more selected from the group consisting of polybutene and liquid paraffin.

### [Aspect 32]

The Present patch according to any one of the Aspects 22 to 31, wherein the contained amount of the base polymer is 5 to 50% by weight, the contained amount of the tackifier resin is 10 to 60% by weight, and the contained amount of the softener is 10 to 60% by weight, relative to the pasty preparation weight.

### [Aspect 33]

The Present patch according to any one of the Aspects 22 to 31, wherein the contained amount of the base polymer is 10 to 40% by weight, the contained amount of the tackifier resin is 15 to 50% by weight, and the contained amount of the softener is 20 to 50% by weight, relative to the pasty preparation weight.

### [Aspect 34]

The Present patch according to any one of the Aspects 22 to 31, wherein the contained amount of the base polymer is 10 to 30% by weight, the contained amount of the tackifier resin is 20 to 40% by weight, and the contained amount of the softener is 30 to 45% by weight, relative to the pasty preparation weight.

### [Aspect 35]

The Present patch according to any one of the Aspects 1 to 34 further comprising one or more ingredient(s) selected from the group consisting of L-menthol, a higher fatty acid, a fatty acid ester, and an antioxidant in the pasty preparation.

### [Aspect 36]

The Present patch according to the Aspect 35 , wherein the higher fatty acid is one or more selected from the group consisting of isostearic acid, myristic acid, palmitic acid, stearic acid, oleic acid, linoleic acid, linolenic acid, capric acid, lauric acid, and caproic acid.

### [Aspect 37]

The Present patch according to the Aspect 35, wherein the higher fatty acid is one or more selected from the group consisting of isostearic acid and oleic acid.

### [Aspect 38]

The Present patch according to the Aspect 35, wherein the higher fatty acid is isostearic acid.

### [Aspect 39]

The Present patch according to any one of the Aspect 35 to 38, wherein the fatty acid ester is one or more selected from the group consisting of hexyl laurate, isopropyl myristate, methyl myristate, myristyl myristate, cetyl myristate, octyldodecyl myristate, isopropyl palmitate, cetyl palmitate, isopropyl isostearate, diisobutyl adipate, diisopropyl adipate, dioctyl adipate, diisopropyl sebacate, diethyl sebacate, ethyl oleate, decyl oleate, oleyl oleate, ethyl linoleate, isopropyl linoleate, cetyl lactate, and ethyl lactate.

### [Aspect 40]

The Present patch according to any one of the Aspect 35 to 38, wherein the fatty acid ester is one or more selected from the group consisting of isopropyl myristate, isopropyl palmitate, isopropyl isostearate, diisopropyl adipate, and diethyl sebacate.

### [Aspect 41]

The Present patch according to any one of the Aspect 35 to 38, wherein the fatty acid ester is diethyl sebacate.

### [Aspect 42]

The Present patch according to any one of the Aspect 35 to 41, wherein the antioxidant is one or more selected from the group consisting of dibutylhydroxytoluene, pentaerythrityl-tetrakis-[3-(3,5-di-t-butyl-4-hydroxyphenyl)propionate], tocopherol acetate, and ascorbic acid.

### [Aspect 43]

The Present patch according to any one of the Aspect 35 to 41, wherein the antioxidant is dibutylhydroxytoluene.

### [Aspect 44]

The Present patch according to the Aspect 35, wherein
the higher fatty acid is isostearic acid;
the fatty acid ester is diethyl sebacate; and
the antioxidant is dibutylhydroxytoluene.

### [Aspect 45]

The Present patch according to any one of the Aspect 35 to 44, wherein the contained amount of the L-menthol is 0.1 to 10% by weight, the contained amount of the higher fatty acid is 0.1 to 20% by weight, the contained amount of the fatty acid ester is 0.1 to 20% by weight, and the contained amount of the antioxidant is 0.05 to 5% by weight, relative to the pasty preparation weight.

### [Aspect 46]

The Present patch according to any one of the Aspect 35 to 44, wherein the contained amount of the L-menthol is 0.5 to 8% by weight, the contained amount of the higher fatty acid is 0.5 to 15% by weight, the contained amount of the fatty acid ester is 0.5 to 15% by weight, and the contained amount of the antioxidant is 0.1 to 1% by weight, relative to the pasty preparation weight.

### [Aspect 47]

The Present patch according to any one of the Aspect 35 to 44, wherein the contained amount of the L-menthol is 1 to 5% by weight, the contained amount of the higher fatty acid is 1 to 10% by weight, the contained amount of the fatty acid ester is 1 to 10% by weight, and the contained amount of the antioxidant is 0.2 to 0.5% by weight, relative to the pasty preparation weight.

### [Aspect 48]

The Present patch according to any one of the Aspects 1 to 47 further comprising one or more ingredient(s) selected from the group consisting of a cellulose derivative, a silicon compound, an inorganic filler, a preservative, a fungicide, a flavoring agent, and a colorant in the pasty preparation.

### [Aspect 49]

The Present patch according to the Aspect 48, wherein
the cellulose derivative is one or more selected from the group consisting of polyvinylpyrrolidone, polyvinyl alcohol, and hydroxypropyl methylcellulose;
the silicon compound is one or more selected from the group consisting of anhydrous silicic acid and light anhydrous silicic acid;
the inorganic filler is one or more selected from the group consisting of silica and zinc stearate;
the preservative is a p-hydroxybenzoate;
the fungicide is one or more selected from the group consisting of ethanol and isopropyl alcohol;
the flavoring agent is mentha oil; and
the colorant is yellow ferric oxide.

### [Aspect 50]

The Present patch comprising loxoprofen sodium dihydrate, lactic acid, zinc oxide, a styrene-isoprene-styrene block copolymer, an alicyclic saturated hydrocarbon resin, polybutene, liquid paraffin, L-menthol, isostearic acid, and dibutylhydroxytoluene in the pasty preparation.

### [Aspect 51]

The Present patch, wherein the pasty preparation consists of loxoprofen sodium dihydrate, lactic acid, zinc oxide, a styrene-isoprene-styrene block copolymer, an alicyclic saturated hydrocarbon resin, polybutene, liquid paraffin, L-menthol, isostearic acid, and dibutylhydroxytoluene.

### [Aspect 52]

The Present patch according to the Aspect 50 or 51, comprising
0.1 to 10% by weight of loxoprofen sodium dihydrate;
0.1 to 2.0% by weight of lactic acid;
0.03 to 1.5% by weight of zinc oxide;
5 to 50% by weight of a styrene-isoprene-styrene block copolymer;
10 to 60% by weight of an alicyclic saturated hydrocarbon resin;
10 to 60% by weight of one or more softener(s) selected from the group consisting of polybutene and liquid paraffin;
0.1 to 10% by weight of L-menthol;
0.1 to 20% by weight of isostearic acid; and
0.05 to 5% by weight of dibutylhydroxytoluene, relative to the pasty preparation weight.

### [Aspect 53]

The Present patch according to the Aspect 50 or 51, comprising
0.5 to 8% by weight of loxoprofen sodium dihydrate;
0.1 to 1.7% by weight of lactic acid;
0.06 to 1.2% by weight of zinc oxide;
10 to 40% by weight of a styrene-isoprene-styrene block copolymer;
15 to 50% by weight of an alicyclic saturated hydrocarbon resin;
20 to 50% by weight of one or more softener(s) selected from the group consisting of polybutene and liquid paraffin;
0.5 to 8% by weight of L-menthol;
0.5 to 15% by weight of isostearic acid; and
0.1 to 1% by weight of dibutylhydroxytoluene, relative to the pasty preparation weight.

### [Aspect 54]

The Present patch according to the Aspect 50 or 51, comprising
1 to 6% by weight of loxoprofen sodium dihydrate;
0.1 to 1.5% by weight of lactic acid;
0.1 to 1.0% by weight of zinc oxide;
10 to 30% by weight of a styrene-isoprene-styrene block copolymer;
20 to 40% by weight of an alicyclic saturated hydrocarbon resin;
30 to 45% by weight of one or more softener(s) selected from the group consisting of polybutene and liquid paraffin;
1 to 5% by weight of L-menthol;
1 to 10% by weight of isostearic acid; and
0.2 to 0.5% by weight of dibutylhydroxytoluene, relative to the pasty preparation weight.

### [Aspect 55]

The Present patch according to any one of the Aspects 52 to 54, wherein the weight ratio of the contained amount of the loxoprofen sodium dihydrate and the contained amount of the lactic acid is 1 : 0.01 to 1 : 0.4.

### [Aspect 56]

The Present patch according to any one of the Aspects 52 to 54, wherein the weight ratio of the contained amount of the loxoprofen sodium dihydrate and the contained amount of the lactic acid is 1 : 0.05 to 1 : 0.35.

### [Aspect 57]

The Present patch according to any one of the Aspects 52 to 54, wherein the weight ratio of the contained amount of the loxoprofen sodium dihydrate and the contained amount of the lactic acid is 1 : 0.07 to 1 : 0.3.

### [Aspect 58]

The Present patch according to any one of the Aspects 52 to 57, wherein the weight ratio of the contained amount of the loxoprofen sodium dihydrate and the contained amount of the zinc oxide is 1 : 0.03 to 1 : 0.3.

### [Aspect 59]

The Present patch according to any one of the Aspects 52 to 57, wherein the weight ratio of the contained amount of the loxoprofen sodium dihydrate and the contained amount of the zinc oxide is 1 : 0.04 to 1 : 0.3.

### [Aspect 60]

The Present patch according to any one of the Aspects 52 to 57, wherein the weight ratio of the contained amount of the loxoprofen sodium dihydrate and the contained amount of the zinc oxide is 1 : 0.05 to 1 : 0.3.

### 2. Method for producing patch

### [Aspect 61]

A method for producing the patch according to any one of the Aspects 1 to 60, comprising
Step 1: dissolving a loxoprofen component in a C₂-C₆ hydroxy acid-containing solution to obtain a loxoprofen-containing solution; and
Step 2: mixing the loxoprofen-containing solution obtained in Step 1 and a metal oxide to obtain a drug liquid.

### [Aspect 62]

The method according to the Aspect 61, wherein the C₂-C₆ hydroxy acid-containing solution comprises a C₂-C₆ hydroxy acid and one or more ingredient(s) selected from the group consisting of L-menthol, a higher fatty acid, and a fatty acid ester.

### [Aspect 63]

The method according to the Aspect 61 or 62 further comprising
Step 3: mixing one or more other ingredient(s) selected from the group consisting of a base polymer, a tackifier resin, a softener, and an antioxidant to obtain an adhesive solution;
Step 4: mixing the drug liquid obtained in Step 2 and the adhesive solution obtained in Step 3 to obtain an adhesive composition;
Step 5: coating a release liner with the adhesive composition obtained in Step 4 to form an adhesive layer; and
Step 6: applying the adhesive layer obtained in Step 5 on a backing to obtain a laminate.

### [Aspect 64]

The method according to any one of the Aspects 61 to 63, comprising
Step 1: mixing loxoprofen sodium dihydrate, lactic acid, and optionally one or more ingredient(s) selected from the group consisting of L-menthol, isostearic acid, and diethyl sebacate to obtain a loxoprofen-containing solution;
Step 2: mixing the loxoprofen-containing solution obtained in Step 1 and zinc oxide to obtain a drug liquid;
Step 3: heating and dissolving a styrene-isoprene-styrene block copolymer, an alicyclic saturated hydrocarbon resin, polybutene and/or liquid paraffin, and dibutylhydroxytoluene to obtain an adhesive solution;
Step 4: mixing the drug liquid obtained in Step 2 and the adhesive solution obtained in Step 3 to obtain an adhesive composition;
Step 5: coating a release liner with the adhesive composition obtained in Step 4 to form an adhesive layer; and
Step 6: applying the adhesive layer obtained in Step 5 on a backing to obtain a laminate.

### [Aspect 65]

A Present patch produced by the method according to any one of the Aspects 61 to 64.

### 3. Use of patch

### [Aspect 66]

The Present patch according to any one of the Aspects 1 to 60 or 65 for preventing or treating inflammation or pain.

### [Aspect 67]

Use of the Present patch according to any one of the Aspects 1 to 60 or 65 in the manufacture of a medicament for preventing or treating inflammation or pain.

### [Aspect 68]

A method for preventing or treating inflammation or pain, which comprises administering the Present patch according to any one of the Aspects 1 to 60 or 65 to a patient.

### [Aspect 69]

Use of the Present patch according to any one of the Aspects 1 to 60 or 65 in the prevention or treatment of inflammation or pain.

### EXAMPLES

Hereinafter, the present invention is more specifically illustrated by means of Examples, but the present invention is not limited to the following Examples. In the Examples (Ex.) and Comparative Examples (Com.), numerical values are expressed in "% by weight", unless otherwise specified.

### [Example 1S]

Loxoprofen sodium dihydrate (hereinafter also referred to as "loxoprofen Na•2H₂O"), lactic acid, and isostearic acid were mixed, dissolution of loxoprofen sodium was visually confirmed, and then zinc oxide was added thereto to prepare a drug liquid.

The contained amount of each ingredient is shown in Table 1.

### [Example 2S to 7S]

According to each amount shown in Table 1 and Table 2, loxoprofen sodium dihydrate, lactic acid, isostearic acid, and L-menthol were mixed, dissolution of loxoprofen sodium was visually confirmed, and then zinc oxide was added thereto to prepare each drug liquid of Examples 2S to 7S.

### [Example 8S]

Loxoprofen sodium dihydrate and lactic acid were mixed, dissolution of loxoprofen sodium was visually confirmed, and then zinc oxide was added thereto to prepare a drug liquid.

The contained amount of each ingredient is shown in Table 2.

**Table 1**

| | Ex. 1S | Ex. 2S | Ex. 3S | Ex. 4S |
|---|---|---|---|---|
| Loxoprofen Na•2H₂O | 5.67 | 5.67 | 5.67 | 5.67 |
| Lactic acid | 0.5 | 0.8 | 0.8 | 0.8 |
| Zinc oxide | 0.5 | 0.5 | 0.5 | 0.5 |
| Isostearic acid | 6 | 2 | 5 | 10 |
| L-Menthol | - | 2.7 | 2.7 | 2.7 |
| Dissolution state | ○ | ○ | ○ | ○ |

**Table 2**

| | Ex. 5S | Ex. 6S | Ex. 7S | Ex. 8S |
|---|---|---|---|---|
| Loxoprofen Na•2H₂O | 5.67 | 5.67 | 5.67 | 5.67 |
| Lactic acid | 0.4 | 1.2 | 1.6 | 1.7 |
| Zinc oxide | 0.5 | 0.5 | 0.5 | 0.5 |
| Isostearic acid | 5 | 5 | 5 | - |
| L-Menthol | 2.7 | 2.7 | 2.7 | - |
| Dissolution state | ○ | ○ | ○ | ○ |

### [Comparative Example 1S]

Lactic acid, isostearic acid, and zinc oxide were mixed, and then loxoprofen sodium dihydrate was added thereto to prepare a drug liquid.

The contained amount of each ingredient is shown in Table 3.

### [Comparative Examples 2S to 7S]

According to each amount shown in Table 3 and Table 4, lactic acid, isostearic acid, zinc oxide, and L-menthol were mixed, and then loxoprofen sodium dihydrate was added thereto to prepare each drug liquid of Comparative Examples 2S to 7S.

### [Comparative Examples 8S to 10S]

Loxoprofen sodium dihydrate, lactic acid, and isostearic acid were mixed, dissolution of loxoprofen sodium was visually confirmed, and then zinc oxide was added thereto to prepare each drug liquid of Comparative Examples 8S to 10S in the same manner as Example 1S except that Comparative Example 8S did not comprise lactic acid and Comparative Example 10S did not comprise isostearic acid.

The contained amount of each ingredient is shown in Table 4.

**Table 3**

| | Com. 1S | Com. 2S | Com. 3S | Com. 4S | Com. 5S |
|---|---|---|---|---|---|
| Loxoprofen Na•2H₂O | 5.67 | 5.67 | 5.67 | 5.67 | 5.67 |
| Lactic acid | 0.5 | 0.8 | 0.8 | 0.8 | 0.4 |
| Zinc oxide | 0.5 | 0.5 | 0.5 | 0.5 | 0.5 |
| Isostearic acid | 6 | 2 | 5 | 10 | 5 |
| L-Menthol | - | 2.7 | 2.7 | 2.7 | 2.7 |
| Dissolution state | × | × | × | × | × |

**Table 4**

| | Com. 6S | Com. 7S | Com. 8S | Com. 9S | Com. 10S |
|---|---|---|---|---|---|
| Loxoprofen Na•2H₂O | 5.67 | 5.67 | 5.67 | 5.67 | 5.67 |
| Lactic acid | 1.2 | 1.6 | - | 2.5 | 2.5 |
| Zinc oxide | 0.5 | 0.5 | 0.5 | 0.5 | 0.5 |
| Isostearic acid | 5 | 5 | 6 | 6 | - |
| L-Menthol | 2.7 | 2.7 | - | - | - |
| Dissolution state | × | × | × | × | × |

### [Example 9]

Loxoprofen sodium dihydrate, lactic acid, and isostearic acid were mixed, dissolution of loxoprofen sodium was visually confirmed, and then zinc oxide was added thereto to prepare a drug liquid.

The ingredients other than the drug liquid, i.e., a SIS (SIS5002; manufactured by JSR Corporation), an alicyclic saturated hydrocarbon resin (ARKON P100; manufactured by Arakawa Chemical Industries, Ltd.), liquid paraffin (HICALL M-352; manufactured by KANEDA Co., Ltd.), and BHT were stirred and dissolved under heating under nitrogen atmosphere to obtain an adhesive solution. To the adhesive solution was added the drug liquid, and the resulting mixture was further stirred and mixed to prepare an adhesive composition. A siliconized polyethylene terephthalate film was coated with the resulting adhesive composition to form an adhesive layer having a thickness of about 150 µm. The resulting adhesive layer was applied on a polyester woven fabric as a backing to obtain a laminate and prepare a patch of Example 9.

The contained amount of each ingredient is shown in Table 5.

### [Comparative Example 11]

Lactic acid, isostearic acid, and zinc oxide were mixed, and then loxoprofen sodium dihydrate was added thereto to prepare a drug liquid.

The ingredients of a SIS (SIS5002; manufactured by JSR Corporation), an alicyclic saturated hydrocarbon resin (ARKON P100; manufactured by Arakawa Chemical Industries, Ltd.), liquid paraffin (HICALL M-352; manufactured by KANEDA Co., Ltd.), and BHT were stirred and dissolved under heating under nitrogen atmosphere to obtain an adhesive solution. To the adhesive solution was added the drug liquid, and the resulting mixture was further stirred and mixed to prepare an adhesive composition. A siliconized polyethylene terephthalate film was coated with the resulting adhesive composition to form an adhesive layer having a thickness of about 150 µm. The resulting adhesive layer was applied on a polyester woven fabric as a backing to obtain a laminate and prepare a patch of Comparative Example 11.

The contained amount of each ingredient is shown in Table 5.

**Table 5**

| | Ex. 9 | Com. 11 |
|---|---|---|
| Loxoprofen Na•2H₂O | 5.67 | 5.67 |
| Isostearic acid | 6 | 6 |
| Lactic acid | 0.5 | 0.5 |
| Zinc oxide | 0.5 | 0.5 |
| SIS | 14 | 14 |
| Liquid paraffin | 38.03 | 38.03 |
| BHT | 0.3 | 0.3 |
| Alicyclic saturated hydrocarbon resin | 35 | 35 |
| Dissolution state of drug liquid | ○ | × |
| Produced amount of ring-opened form of loxoprofen (%) (60°C/2 weeks) | 0.722 | 0.797 |
| Cumulative permeation amount after 12 hours (µg/cm²) | 44.13 | 30.13 |

### [Examples 10 and 11]

Each patch of Examples 10 and 11 was obtained according to the same method as Example 9 using the contained amounts shown in Table 6, except that to the adhesive solution was added polybutene and to the drug liquid was added L-menthol.

### [Example 12]

The patch of Example 12 was obtained according to the same method as Example 9 using the contained amounts shown in Table 6, except that diethyl sebacate was used instead of isostearic acid.

### [Comparative Example 12]

The patch of Comparative Example 12 was obtained according to the same method as Example 9 using the contained amounts shown in Table 6, except that to the adhesive solution was added polybutene, to the drug liquid was added L-menthol, and zinc oxide was not contained.

**Table 6**

| | Ex. 10 | Ex. 11 | Ex. 12 | Com. 12 |
|---|---|---|---|---|
| Loxoprofen Na•2H₂O | 5.67 | 5.67 | 2 | 5.67 |
| Lactic acid | 0.7 | 1.2 | 0.5 | 1.8 |
| Zinc oxide | 0.3 | 0.4 | 0.5 | - |
| Isostearic acid | 6 | 4 | - | 6 |
| Diethyl sebacate | - | - | 5 | - |
| L-Menthol | 2.7 | 2.7 | - | 2.7 |
| SIS | 14 | 14 | 14 | 14 |
| Liquid paraffin | 31.33 | 32.73 | 42.7 | 30.53 |
| Polybutene | 2 | 2 | - | 2 |
| BHT | 0.3 | 0.3 | 0.3 | 0.3 |
| Alicyclic saturated hydrocarbon resin | 37 | 37 | 35 | 37 |
| Dissolution state of drug liquid | ○ | ○ | ○ | ○ |
| Produced amount of ring-opened form of loxoprofen (%) | 0.58 | 0.5 | 0.17 | 1.16 |
| (40°C/6 months) | | | | |

### [Test Examples]

### [Test Example 1] Evaluation of dissolution state of drug liquid

The dissolution state of each drug liquid of Examples 1S to 8S and Examples 9 to 12, and Comparative Examples 1S to 10S and Comparative Examples 11 to 12 was visually confirmed. A drug liquid showing a dissolved state (clear state) was indicated as "∘", while a drug liquid showing an undissolved matter (white cloudy state) was indicated as "×".

The test results are shown in Tables 1 to 6.

### [Test Example 2] Quantitative test of ring-opened form of loxoprofen

Each patch of Example 9 and Comparative Example 11 stored under a storage condition of 60°C for 2 weeks, and Examples 10 to 12 and Comparative Example 12 stored under a storage condition of 40°C for 6 months was cut into a size of 5 × 7 cm², put into a 50mL centrifuge tube, an internal standard substance and tetrahydrofuran (hereinafter referred to as "THF") were added thereto, and the patch was subjected to ultrasonic extraction and extraction with a shaker. A 0.5 mL of said extract was taken, and a 40% acetonitrile aqueous solution was added thereto such that the volume became 5 mL. Then, the resulting solution was filtered through a membrane filter (0.45 µm), and the produced amount of ring-opened form of loxoprofen was measured by HPLC method using the following measurement conditions.

The test results are shown in Tables 5 and 6. In this regard, the produced amount of ring-opened form of loxoprofen is shown as the ratio of peak area of ring-opened form of loxoprofen (%) relative to peak area of the drug ingredient (loxoprofen sodium).

### [HPLC measurement conditions]

- Column: ACQUITY UPLC BEH C8 (2.1 × 100 mm)
- Mobile phase: acetonitrile : water : phosphoric acid = 280 : 720 : 1
- Wavelength: 222 nm
- Flow rate: 0.4 mL/min

### [Test Example 3] In vitro skin permeability test

Each patch of Example 9 and Comparative Example 11 was subjected to an in vitro hairless rat skin permeability test. An excised abdominal skin of a male hairless rat (HWY strain, 7 weeks old) was put in a Franz diffusion cell. The dermis side was set to be the receptor side, the inside of the receptor side was filled with phosphate buffered saline, and hot water of 37°C was circulated in the water jacket. Each patch was cut into a round shape (1.54 cm²), and applied to the excised skin. The receptor solution was sampled after the test start until 12 hours, and the amount of the drug permeated the skin was measured by HPLC method using the following measurement conditions.

The test results are shown in Table 5.

### [HPLC measurement conditions]

- Column: ACQUITY UPLC BEH C18 (2.1 × 50 mm)
- Mobile phase: acetonitrile : water : phosphoric acid = 420 : 580 : 1
- Wavelength: 222 nm
- Flow rate: 0.4 mL/min

As can be understood from the results of Examples 1S to 8S and Example 9, and Comparative Examples 1S to 7S and Comparative Example 11, when loxoprofen sodium dihydrate and lactic acid were mixed at first and then zinc oxide was added thereto, the resulting drug liquid showed a dissolution state. Meanwhile, when lactic acid and zinc oxide were mixed at first and then loxoprofen sodium dihydrate was added thereto, undissolved matters were observed and a homogeneous drug liquid could not be prepared.

Also, as can be understood from the comparison of Example 9 with Comparative Example 11, the patch prepared by mixing loxoprofen sodium dihydrate and lactic acid at first and then adding zinc oxide thereto reduced the produced amount of ring-opened form of loxoprofen, which is a degradation product specific to loxoprofen, to about 90%, and yet showed a high cumulative permeation amount of the drug of about 146% at 12 hours, as compared to the patch prepared by mixing lactic acid and zinc oxide at first and then adding loxoprofen sodium dihydrate thereto. Thus, the Present patch was illustrated to be a formulation which can suppress the degradation of the drug and show excellent skin permeability.

Also, as can be understood from the results of Comparative Examples 8S to 10S, even when loxoprofen sodium dihydrate and lactic acid were mixed at first and then zinc oxide was added thereto, if the contained amount of lactic acid was insufficient or the contained amount of lactic acid was too much, undissolved matters were observed in the drug liquid and a homogeneous drug liquid could not be prepared.

Further, as can be understood from the results of Examples 10 to 12 and Comparative Example 12, Examples 10 to 12 comprising zinc oxide significantly suppressed the produced amount of ring-opened form of loxoprofen, as compared to Comparative Example 12 comprising no zinc oxide.

### INDUSTRIAL APPLICABILITY

The present invention can provide a patch which shows excellent drug releasability and suppresses the generation of ring-opened form of loxoprofen which is a degradation product specific to loxoprofen and produced in a formulation during storage or manufacturing process. Also, because an undissolved matter of a drug liquid is not generated at the time of manufacture according to the present invention, the present invention can provide a method for producing the patch having excellent manufacturing properties such as improvement in the workability during the preparation of a drug liquid and ease in homogeneously containing the drug in a pasty preparation.

## Claims

1. A patch comprising loxoprofen or a salt thereof, or a hydrate thereof; lactic acid as the sole hydroxy acid; and zinc oxide as the sole metal oxide in a pasty preparation, wherein
the loxoprofen or a salt thereof, or a hydrate thereof is loxoprofen sodium or a hydrate thereof; and
the contained amount of the loxoprofen or a salt thereof, or a hydrate thereof is 0.1 to 10% by weight, the contained amount of the lactic acid is 0.1 to 2.0% by weight, and the contained amount of the zinc oxide is 0.03 to 1.5% by weight, relative to the pasty preparation weight.

2. The patch according to claim 1, wherein
the loxoprofen or a salt thereof, or a hydrate thereof is loxoprofen sodium dihydrate.

3. The patch according to any one of claims 1 to 2, wherein the weight ratio of the contained amount of the loxoprofen or a salt thereof, or a hydrate thereof and the contained amount of the lactic acid is 1 : 0.01 to 1 : 0.4.

4. The patch according to any one of claims 1 to 3, wherein the weight ratio of the contained amount of the loxoprofen or a salt thereof, or a hydrate thereof and the contained amount of the zinc oxide is 1 : 0.03 to 1 : 0.3.

5. The patch according to any one of claims 1 to 4 further comprising one or more ingredient(s) selected from the group consisting of a base polymer, a tackifier resin, and a softener in the pasty preparation.

6. The patch according to claim 5, wherein
the base polymer is a styrene-isoprene-styrene block copolymer;
the tackifier resin is an alicyclic saturated hydrocarbon resin; and
the softener is one or more selected from the group consisting of polybutene and liquid paraffin.

7. The patch according to claim 5 or **6,** wherein the contained amount of the base polymer is 5 to 50% by weight, the contained amount of the tackifier resin is 10 to 60% by weight, and the contained amount of the softener is 10 to 60% by weight, relative to the pasty preparation weight.

8. The patch according to any one of claims 1 to 7 further comprising one or more ingredient(s) selected from the group consisting of L-menthol, a higher fatty acid, a fatty acid ester, and an antioxidant in the pasty preparation.

9. The patch according to claim 8, wherein
the higher fatty acid is isostearic acid;
the fatty acid ester is diethyl sebacate; and
the antioxidant is dibutylhydroxytoluene.

10. The patch according to claim 8 or 9, wherein the contained amount of the L-menthol is 0.1 to 10% by weight, the contained amount of the higher fatty acid is 0.1 to 20% by weight, the contained amount of the fatty acid ester is 0.1 to 20% by weight, and the contained amount of the antioxidant is 0.05 to 5% by weight, relative to the pasty preparation weight.

11. The patch according to any one of claims 1 to 8 comprising loxoprofen sodium dihydrate, lactic acid, zinc oxide, a styrene-isoprene-styrene block copolymer, an alicyclic saturated hydrocarbon resin, polybutene, liquid paraffin, L-menthol, isostearic acid, and dibutylhydroxytoluene in a pasty preparation.

12. A method for producing the patch according to any one of claims 1 to 11, comprising
Step 1: dissolving loxoprofen or a salt thereof, or a hydrate thereof in lactic acid-containing solution to obtain a loxoprofen-containing solution; and
Step 2: mixing the loxoprofen-containing solution obtained in Step 1 and zinc oxide to obtain a drug liquid.

13. The method according to claim 12, wherein the lactic acid-containing solution comprises lactic acid and one or more ingredient(s) selected from the group consisting of L-menthol, a higher fatty acid, and a fatty acid ester.

## Patentansprüche

1. Pflaster, umfassend Loxoprofen oder ein Salz davon oder ein Hydrat davon; Milchsäure als einzige Hydroxysäure; und Zinkoxid als einziges Metalloxid in einer pastösen Zubereitung, wobei
das Loxoprofen oder ein Salz davon oder ein Hydrat davon Loxoprofen-Natrium oder ein Hydrat davon ist; und
die enthaltene Menge des Loxoprofens oder eines Salzes davon oder eines Hydrats davon 0,1 bis 10 Gew.-% beträgt, die enthaltene Menge der Milchsäure 0,1 bis 2,0 Gew.-% beträgt und die enthaltene Menge des Zinkoxids 0,03 bis 1,5 Gew.-% beträgt, bezogen auf das Gewicht der pastösen Zubereitung.

2. Pflaster nach Anspruch 1, wobei
das Loxoprofen oder ein Salz davon oder ein Hydrat davon Loxoprofen-Natrium-Dihydrat ist.

3. Pflaster nach einem der Ansprüche 1 bis 2, wobei das Gewichtsverhältnis der enthaltenen Menge des Loxoprofens oder eines Salzes davon oder eines Hydrats davon zu der enthaltenen Menge der Milchsäure 1 : 0,01 bis 1 : 0,4 beträgt.

4. Pflaster nach einem der Ansprüche 1 bis 3, wobei das Gewichtsverhältnis der enthaltenen Menge des Loxoprofens oder eines Salzes davon oder eines Hydrats davon zu der enthaltenen Menge des Zinkoxids 1 : 0,03 bis 1 : 0,3 beträgt.

5. Pflaster nach einem der Ansprüche 1 bis 4, ferner umfassend einen oder mehrere Bestandteile, ausgewählt aus der Gruppe bestehend aus einem Basispolymer, einem Klebrigmacherharz und einem Weichmacher, in der pastösen Zubereitung.

6. Pflaster nach Anspruch 5, wobei
das Basispolymer ein Styrol-Isopren-Styrol-Blockcopolymer ist;
das Klebrigmacherharz ein alicyclisches gesättigtes Kohlenwasserstoffharz ist; und
der Weichmacher einer oder mehrere ist, ausgewählt aus der Gruppe bestehend aus Polybuten und flüssigem Paraffin.

7. Pflaster nach Anspruch 5 oder 6, wobei die enthaltene Menge des Basispolymers 5 bis 50 Gew.-% beträgt, die enthaltene Menge des Klebrigmacherharzes 10 bis 60 Gew.-% beträgt und die enthaltene Menge des Weichmachers 10 bis 60 Gew.-% beträgt, bezogen auf das Gewicht der pastösen Zubereitung.

8. Pflaster nach einem der Ansprüche 1 bis 7, ferner umfassend einen oder mehrere Bestandteile, ausgewählt aus der Gruppe bestehend aus L-Menthol, einer höheren Fettsäure, einem Fettsäureester und einem Antioxidationsmittel, in der pastösen Zubereitung.

9. Pflaster nach Anspruch 8, wobei
die höhere Fettsäure Isostearinsäure ist;
der Fettsäureester Diethylsebacat ist; und
das Antioxidationsmittel Dibutylhydroxytoluol ist.

10. Pflaster nach Anspruch 8 oder 9, wobei die enthaltene Menge des L-Menthols 0,1 bis 10 Gew.-% beträgt, die enthaltene Menge der höheren Fettsäure 0,1 bis 20 Gew.-% beträgt, die enthaltene Menge des Fettsäureesters 0,1 bis 20 Gew.-% beträgt und die enthaltene Menge des Antioxidationsmittels 0,05 bis 5 Gew.-% beträgt, bezogen auf das Gewicht der pastösen Zubereitung.

11. Pflaster nach einem der Ansprüche 1 bis 8, umfassend Loxoprofen-Natrium-Dihydrat, Milchsäure, Zinkoxid, ein Styrol-Isopren-Styrol-Blockcopolymer, ein alicyclisches gesättigtes Kohlenwasserstoffharz, Polybuten, flüssiges Paraffin, L-Menthol, Isostearinsäure und Dibutylhydroxytoluol in einer pastösen Zubereitung.

12. Verfahren zur Herstellung des Pflasters nach einem der Ansprüche 1 bis 11, umfassend
| | |
|---|---|
| Schritt 1: | Lösen von Loxoprofen oder einem Salz davon oder einem Hydrat davon in einer milchsäurehaltigen Lösung, um eine loxoprofenhaltige Lösung zu erhalten; und |
| Schritt 2: | Mischen der in Schritt 1 erhaltenen loxoprofenhaltigen Lösung und von Zinkoxid, um eine Wirkstoffflüssigkeit zu erhalten. |

13. Verfahren nach Anspruch 12, wobei die milchsäurehaltige Lösung Milchsäure und einen oder mehrere Bestandteile, ausgewählt aus der Gruppe bestehend aus L-Menthol, einer höheren Fettsäure und einem Fettsäureester, umfasst.

## Revendications

1. Emplâtre comprenant du loxoprofène ou un sel de celui-ci, ou un hydrate de celui-ci ; de l'acide lactique en tant que seul acide hydroxylé ; et de l'oxyde de zinc en tant que seul oxyde métallique dans une préparation pâteuse, dans lequel
le loxoprofène ou un sel de celui-ci, ou un hydrate de celui-ci est le loxoprofène sodique ou un hydrate de celui-ci ; et
la quantité contenue de loxoprofène ou d'un sel de celui-ci, ou d'un hydrate de celui-ci est de 0,1 à 10 % en poids, la quantité contenue d'acide lactique est de 0,1 à 2,0 % en poids et la quantité contenue d'oxyde de zinc est de 0,03 à 1,5 % en poids, par rapport au poids de la préparation pâteuse.

2. Emplâtre selon la revendication 1, dans lequel
le loxoprofène ou un sel de celui-ci, ou un hydrate de celui-ci est le dihydrate de loxoprofène sodique.

3. Emplâtre selon l'une quelconque des revendications 1 à 2, dans lequel le rapport pondéral entre la quantité contenue de loxoprofène ou d'un sel de celui-ci, ou d'un hydrate de celui-ci et la quantité contenue d'acide lactique est de 1 : 0,01 à 1 : 0,4.

4. Emplâtre selon l'une quelconque des revendications 1 à 3, dans lequel le rapport pondéral entre la quantité contenue de loxoprofène ou d'un sel de celui-ci, ou d'un hydrate de celui-ci et la quantité contenue d'oxyde de zinc est de 1 : 0,03 à 1 : 0,3.

5. Emplâtre selon l'une quelconque des revendications 1 à 4, comprenant en outre un ou plusieurs ingrédients choisis dans le groupe constitué par un polymère de base, une résine tackifiante et un plastifiant, dans la préparation pâteuse.

6. Emplâtre selon la revendication 5, dans lequel
le polymère de base est un copolymère séquencé styrène-isoprène-styrène ;
la résine tackifiante est une résine hydrocarbonée saturée alicyclique ; et
le plastifiant est un ou plusieurs choisis dans le groupe constitué par le polybutène et la paraffine liquide.

7. Emplâtre selon la revendication 5 ou 6, dans lequel la quantité contenue de polymère de base est de 5 à 50 % en poids, la quantité contenue de résine tackifiante est de 10 à 60 % en poids et la quantité contenue de plastifiant est de 10 à 60 % en poids, par rapport au poids de la préparation pâteuse.

8. Emplâtre selon l'une quelconque des revendications 1 à 7, comprenant en outre un ou plusieurs ingrédients choisis dans le groupe constitué par le L-menthol, un acide gras supérieur, un ester d'acide gras et un antioxydant, dans la préparation pâteuse.

9. Emplâtre selon la revendication 8, dans lequel
l'acide gras supérieur est l'acide isostéarique ;
l'ester d'acide gras est le sébacate de diéthyle ; et
l'antioxydant est le dibutylhydroxytoluène.

10. Emplâtre selon la revendication 8 ou 9, dans lequel la quantité contenue de L-menthol est de 0,1 à 10 % en poids, la quantité contenue d'acide gras supérieur est de 0,1 à 20 % en poids, la quantité contenue d'ester d'acide gras est de 0,1 à 20 % en poids et la quantité contenue d'antioxydant est de 0,05 à 5 % en poids, par rapport au poids de la préparation pâteuse.

11. Emplâtre selon l'une quelconque des revendications 1 à 8, comprenant du dihydrate de loxoprofène sodique, de l'acide lactique, de l'oxyde de zinc, un copolymère séquencé styrène-isoprène-styrène, une résine hydrocarbonée saturée alicyclique, du polybutène, de la paraffine liquide, du L-menthol, de l'acide isostéarique et du dibutylhydroxytoluène dans une préparation pâteuse.

12. Procédé de production de l'emplâtre selon l'une quelconque des revendications 1 à 11, comprenant
| | |
|---|---|
| Étape 1 : | la dissolution de loxoprofène ou d'un sel de celui-ci, ou d'un hydrate de celui-ci dans une solution contenant de l'acide lactique pour obtenir une solution contenant du loxoprofène ; et |
| Étape 2 : | le mélange de la solution contenant du loxoprofène obtenue à l'étape 1 et d'oxyde de zinc pour obtenir un liquide médicamenteux. |

13. Procédé selon la revendication 12, dans lequel la solution contenant de l'acide lactique comprend de l'acide lactique et un ou plusieurs ingrédients choisis dans le groupe constitué par le L-menthol, un acide gras supérieur et un ester d'acide gras.
